# EUROPEAN PATENT SPECIFICATION

(11) **EP 4 215 118 B1**
(45) Date of publication and mention of the grant of the patent: **09.10.2024**
(21) Application number: 23151978.6
(22) Date of filing: 17.01.2023
(51) Int. Cl.: A61B 6/02, A61B 6/00, G06T 7/00

(54) **IMAGE PROCESSING APPARATUS, IMAGE PROCESSING METHOD, AND PROGRAM**
BILDVERARBEITUNGSVORRICHTUNG, BILDVERARBEITUNGSVERFAHREN UND PROGRAMM
APPAREIL DE TRAITEMENT D'IMAGE, PROCÉDÉ DE TRAITEMENT D'IMAGE ET PROGRAMME

(30) Priority: 19.01.2022 JP 2022006670
(43) Date of publication of application: 26.07.2023
(73) Proprietor: FUJIFILM Corporation, Tokyo 106-8620 (JP)
(72) Inventor: MACHII, Yusuke, Kanagawa, 258-8538 (JP)
(74) Representative: Dehns Germany Partnerschaft mbB

(56) References cited:
- WO-A1-2011/051863
- US-A1- 2016 249 868
- WHEELER F W ET AL: "Micro-Calcification Detection in Digital Tomosynthesis Mammography", PROCEEDINGS OF SPIE, IEEE, US, vol. 6144, 13 February 2006 (2006-02-13), pages 614420 - 1, XP002497881, ISBN: 978-1-62841-730-2, DOI: 10.1117/12.653478
- REISER I ET AL: "Automated detection of microcalcification clusters for digital breast tomosynthesis using projection data only: A preliminary study", MEDICAL PHYSICS, AIP, MELVILLE, NY, US, vol. 35, no. 4, 18 March 2008 (2008-03-18), pages 1486 - 1493, XP012116006, ISSN: 0094-2405, DOI: 10.1118/1.2885366

## Description

### BACKGROUND OF THE INVENTION

### 1. Field of the Invention

The present disclosure relates to an image processing apparatus, an image processing method, and a program.

### 2. Description of the Related Art

A technique for recognizing a tissue of a breast in which calcification may occur by using a radiation image obtained by irradiating the breast with radiations is known. For example, JP2016-022143A discloses a technique of specifying pixel regions in which calcification may occur from a radiation image or the like, grouping a set of the specified pixel regions, and displaying the set of the specified pixel regions in color or brightness according to the number of the pixel regions belonging to the group. Thereby, it is possible to intuitively recognize a dense state of a fine calcification tissue.

In addition, tomosynthesis imaging in which a series of a plurality of projection images is acquired by irradiating a breast with radiations having a plurality of angles is known. By reconfiguring the plurality of projection images obtained by tomosynthesis imaging, a plurality of tomographic images in which an overlap of mammary glands is reduced are obtained. Further, a technique of generating one synthesized two-dimensional image in which an overlap of mammary glands is reduced by combining a plurality of tomographic images is known. In addition, JP2020-141867A discloses a technique of generating a two-dimensional image corresponding to the synthesized two-dimensional image by inputting a projection image obtained at a radiation irradiation angle of approximately 0 degree to a learned model instead of the plurality of tomographic images.

F W Wheeler et al: describe in "Micro-Calcification Detection in Digital Tomosynthesis Mammography" (PROCEEDINGS OF SPIE, IEEE, vol. 6144, 13 February 2006) that calcification residual images are computed for each of the projection images, and calcification detection is then performed over 3D space.

### SUMMARY OF THE INVENTION

In image diagnosis for diagnosing calcification of a breast, a shape of a calcification image appearing in a tomographic image, a synthesized two-dimensional image, or the like is important information. However, in the tomographic image, the calcification image is blurred due to noise and visibility is lowered. In addition, in the synthesized two-dimensional image generated based on the plurality of tomographic images, a shape of the calcification image is not accurately represented.

In JP2016-022143A, a distribution state of the calcification image is considered as useful information for image diagnosis for diagnosing calcification. However, a shape of the calcification image is not considered. Further, also in JP2020-141867A, a shape of the calcification image is not considered as useful information for image diagnosis for diagnosing calcification. In image diagnosis for diagnosing calcification, in order to determine whether the calcification image represents malignancy or benignancy, it is desired to accurately determine a type of a shape of the calcification image.

An object of a technique of the present disclosure is to provide an image processing apparatus, an image processing method, and a program capable of accurately determining a type of a shape of a calcification image.

In order to achieve the above object, according to an aspect of the present disclosure, there is provided an image processing apparatus including: at least one processor, in which the processor is configured to execute region-of-interest image generation processing of generating a region-of-interest image from a projection image, which is obtained at an irradiation position closest to a position facing a detection surface of a radiation detector, among a series of projection images obtained by irradiating a breast with radiations and imaging the breast, and shape type determination processing of determining a type of a shape of a calcification image included in the region-of-interest image generated by the region-of-interest image generation processing.

Preferably, the processor is configured to execute calcification image detection processing of detecting the calcification image based on a plurality of tomographic images obtained from a plurality of the projection images, and generate the region-of-interest image in the region-of-interest image generation processing by cutting out a region including the calcification image detected by the calcification image detection processing from the projection image obtained at the irradiation position closest to the position facing the detection surface.

Preferably, the processor is configured to individually generate the region-of-interest image for each of a plurality of the calcification images in the region-of-interest image generation processing in a case where the plurality of calcification images are detected in the calcification image detection processing.

Preferably, the processor is configured to detect only the calcification image of which a signal value is equal to or smaller than a certain value in the calcification image detection processing.

Preferably, the processor is configured to generate a plurality of the region-of-interest images by dividing the projection image obtained at the irradiation position closest to the position facing the detection surface into a plurality of regions in the region-of-interest image generation processing.

Preferably, the processor is configured to execute the shape type determination processing by inputting the region-of-interest image into a machine-learned model obtained by performing machine learning of a relationship between the calcification image and the type of the shape of the calcification image.

Preferably, the processor is configured to determine the shape of the calcification image after performing noise removal processing or resolution enhancement processing, or both the noise removal processing and the resolution enhancement processing on the region-of-interest image in the shape type determination processing.

Preferably, the processor is configured to execute display processing of displaying a shape type determination result by the shape type determination processing on a display unit.

Preferably, the processor is configured to highlight and display the calcification image having a specific shape based on the shape type determination result in the display processing.

Preferably, the processor is configured to determine whether the calcification image represents benignancy or malignancy, or determine a degree of malignancy represented by the calcification image, based on a shape type determination result by the shape type determination processing.

According to another aspect of the present disclosure, there is provided an image processing method including: a region-of-interest image generation step of generating a region-of-interest image from a projection image, which is obtained at an irradiation position closest to a position facing a detection surface of a radiation detector, among a series of projection images obtained by irradiating a breast with radiations and imaging the breast; and a shape type determination step of determining a type of a shape of a calcification image included in the region-of-interest image generated by the region-of-interest image generation step.

According to still another aspect of the present disclosure, there is provided a program causing a computer to execute a process including: region-of-interest image generation processing of generating a region-of-interest image from a projection image, which is obtained at an irradiation position closest to a position facing a detection surface of a radiation detector, among a series of projection images obtained by irradiating a breast with radiations and imaging the breast; and shape type determination processing of determining a type of a shape of a calcification image included in the region-of-interest image generated by the region-of-interest image generation processing.

According to the technique of the present disclosure, it is possible to provide an image processing apparatus, an image processing method, and a program capable of accurately determining a type of a shape of a calcification image.

### BRIEF DESCRIPTION OF THE DRAWINGS

Fig. 1 is a diagram illustrating an example of an entire configuration of a radiography system.
Fig. 2 is a diagram illustrating an example of tomosynthesis imaging.
Fig. 3 is a block diagram illustrating an example of a configuration of an image processing apparatus.
Fig. 4 is a block diagram illustrating an example of a function realized by a controller of the image processing apparatus.
Fig. 5 is a diagram schematically illustrating a flow of processing by the image processing apparatus.
Fig. 6 is a diagram conceptually illustrating region-of-interest image generation processing.
Fig. 7 is a diagram conceptually illustrating learning processing of a machine learning model.
Fig. 8 is a diagram illustrating an example of display processing.
Fig. 9 is a flowchart illustrating a flow of a series of processing by the image processing apparatus.
Fig. 10 is a diagram illustrating a difference in shape of a calcification image due to a difference in irradiation position.
Fig. 11 is a block diagram illustrating a function realized by the controller of the image processing apparatus according to a first modification example.
Fig. 12 is a block diagram schematically illustrating a flow of processing by the image processing apparatus according to the first modification example.
Fig. 13 is a block diagram illustrating a function realized by the controller of the image processing apparatus according to a second modification example.
Fig. 14 is a block diagram schematically illustrating a flow of processing by the image processing apparatus according to the second modification example.
Fig. 15 is a diagram illustrating an example of region-of-interest image generation processing according to a third modification example.
Fig. 16 is a diagram illustrating another example of region-of-interest image generation processing according to the third modification example.

### DESCRIPTION OF THE PREFERRED EMBODIMENTS

Hereinafter, an embodiment of the present disclosure will be described with reference to the drawings.

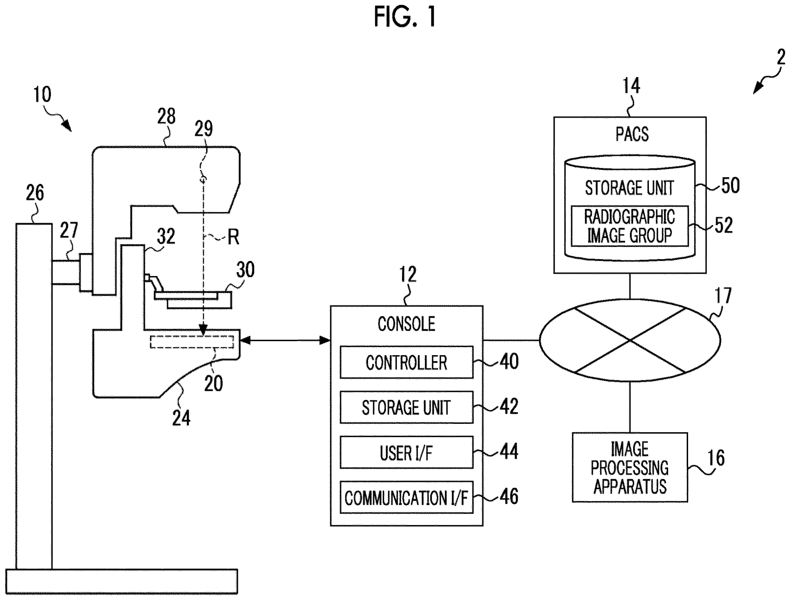

Fig. 1 illustrates an example of an entire configuration of a radiography system 2 according to the present embodiment. The radiography system 2 includes a mammography apparatus 10, a console 12, a picture archiving and communication systems (PACS) 14, and an image processing apparatus 16. The console 12, the PACS 14, and the image processing apparatus 16 are connected to each other via a network 17 by wired communication or wireless communication.

Fig. 1 illustrates an example of an appearance of the mammography apparatus 10. Fig. 1 illustrates an example of an appearance in a case where the mammography apparatus 10 is viewed from a left side of a subject.

The mammography apparatus 10 operates according to a control of the console 12, and is a radiography apparatus that acquires a radiation image of a breast M by irradiating the breast M of the subject as a target with radiations R (for example, X rays) from a radiation source 29.

The mammography apparatus 10 has a function of performing normal imaging in which imaging is performed in a state where the radiation source 29 is positioned at an irradiation position along a normal direction of a detection surface 20A of a radiation detector 20 and a function of performing tomosynthesis imaging in which imaging is performed in a state where the radiation source 29 is moved to each of a plurality of irradiation positions.

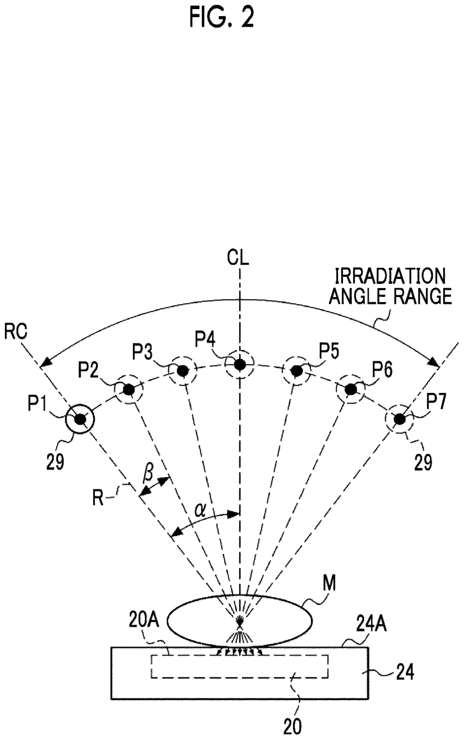

As illustrated in Fig. 1, the mammography apparatus 10 includes an imaging table 24, a base 26, an arm portion 28, and a compression unit 32. A radiation detector 20 is disposed inside the imaging table 24. As illustrated in Fig. 2, in the mammography apparatus 10, in a case of performing imaging, the breast M of the subject is positioned on an imaging surface 24A of the imaging table 24 by a user.

The radiation detector 20 detects radiations R passing through the breast M as a target. Specifically, the radiation detector 20 detects the radiations R that pass through the breast M of the subject, enter into the imaging table 24, and reach a detection surface 20A of the radiation detector 20, and generates a radiation image based on the detected radiations R. The radiation detector 20 outputs image data representing the generated radiation image. In the following, a series of operations of irradiating the breast with radiations R from the radiation source 29 and generating a radiation image by the radiation detector 20 may be referred to as "imaging". The radiation detector 20 may be an indirect-conversion-type radiation detector that converts the radiations R into light beams and converts the converted light beams into charges, or may be a direct-conversion-type radiation detector that directly converts the radiations R into charges.

A compression plate 30 that is used for compressing the breast M when performing imaging is attached to the compression unit 32. The compression plate 30 is moved in a direction toward or away from the imaging table 24 (hereinafter, referred to as a "vertical direction") by a compression plate driving unit (not illustrated) provided in the compression unit 32. The compression plate 30 compresses the breast M between the compression plate 30 and the imaging table 24 by moving in the vertical direction.

The arm portion 28 can be rotated with respect to the base 26 by a shaft portion 27. The shaft portion 27 is fixed to the base 26, and the shaft portion 27 and the arm portion 28 are rotated as one body. Gears are provided in each of the shaft portion 27 and the compression unit 32 of the imaging table 24. By switching the gears between an engaged state and a non-engaged state, the compression unit 32 of the imaging table 24 and the shaft portion 27 can be switched between a state where the compression unit 32 and the shaft portion 27 are connected to each other and are rotated as one body and a state where the shaft portion 27 is separated from the imaging table 24 and idles. Elements for switching between transmission and non-transmission of power of the shaft portion 27 are not limited to the gears, and various mechanical elements can be used. The arm portion 28 and the imaging table 24 can be separately rotated with respect to the base 26 with the shaft portion 27 as a rotation axis.

In a case of performing tomosynthesis imaging in the mammography apparatus 10, the radiation source 29 is sequentially moved to each of a plurality of irradiation positions having different irradiation angles by rotation of the arm portion 28. The radiation source 29 includes a radiation tube (not illustrated) that generates the radiations R, and the radiation tube is moved to each of the plurality of irradiation positions in accordance with the movement of the radiation source 29.

Fig. 2 illustrates an example of tomosynthesis imaging. In Fig. 2, the compression plate 30 is not illustrated. In the present embodiment, the radiation source 29 is moved to irradiation positions Pk (k = 1, 2, ..., 7) at which irradiation angles are different by a certain angle β. That is, the radiation source 29 is sequentially moved to a plurality of positions at which the irradiation angles of the radiations R with respect to the detection surface 20A of the radiation detector 20 are different. In Fig. 2, the number of the irradiation positions Pk is set to 7. On the other hand, the number of the irradiation positions Pk is not limited and can be changed as appropriate.

At each irradiation position Pk, the radiation R is emitted from the radiation source 29 toward the breast M, and the radiation detector 20 generates a radiation image by detecting the radiation R passing through the breast M. In the radiography system 2, in a case where the radiation source 29 is moved to each of the irradiation positions Pk and tomosynthesis imaging for generating a radiation image at each irradiation position Pk is performed, in the example of Fig. 2, seven radiation images are obtained.

In the following, in the tomosynthesis imaging, the radiation image obtained by performing imaging at each irradiation position Pk is referred to as a "projection image" in a case of distinguishing and describing the radiation image from a tomographic image, and a plurality of projection images obtained by performing tomosynthesis imaging once are referred to as a "series of the plurality of projection images". Further, in a case where the projection image is referred to without distinguishing the projection image from the tomographic image, the projection image is simply referred to as a "radiation image".

In addition, as illustrated in Fig. 2, the irradiation angle of the radiation R means an angle α formed by a normal line CL of the detection surface 20A of the radiation detector 20 and a radiation axis RC. The radiation axis RC means an axis connecting a focus of the radiation source 29 at each irradiation position Pk and a preset position. Further, the detection surface 20A of the radiation detector 20 is a surface substantially parallel to the imaging surface 24A. The radiation R emitted from the radiation source 29 is a cone beam having a focus as the apex and the radiation axis RC as a central axis.

On the other hand, in a case of performing normal imaging in the mammography apparatus 10, the position of the radiation source 29 is fixed to the irradiation position P4 at which the irradiation angle α is 0 degree. The radiation R is emitted from the radiation source 29 according to an instruction of the console 12, and the radiation detector 20 generates a radiation image by detecting the radiation R passing through the breast M.

The mammography apparatus 10 and the console 12 are connected to each other by wired communication or wireless communication. The radiation image generated by the radiation detector 20 in the mammography apparatus 10 is output to the console 12 by wired communication or wireless communication via a communication interface (I/F) (not illustrated).

The console 12 includes a controller 40, a storage unit 42, a user I/F 44, and a communication I/F 46. As described above, the controller 40 has a function of performing control related to radiography by the mammography apparatus 10. The controller 40 is configured with, for example, a computer system including a central processing unit (CPU), a read only memory (ROM), and a random access memory (RAM).

The storage unit 42 stores information related to radiography, the radiation image acquired from the mammography apparatus 10, and the like. The storage unit 42 is a non-volatile storage such as a hard disk drive (HDD) or a solid state drive (SSD).

The user I/F 44 includes an input device including various buttons and switches, which are related to imaging of the radiation image and are operated by a user such as a technician, and a lamp, a display, or the like that displays information related to imaging, the radiation image obtained by imaging, and the like.

The communication I/F 46 performs communication of various types of data such as the information related to radiography, the radiation image, and the like between the console 12 and the mammography apparatus 10 by wired communication or wireless communication. Further, the communication I/F 46 performs communication of various types of data such as the radiation image between the PACS 14 and the image processing apparatus 16 via the network 17 by wired communication or wireless communication.

In addition, the PACS 14 includes a storage unit 50 (refer to Fig. 1) that stores a radiation image group 52. The radiation image group 52 includes a projection image acquired from the console 12 via the network 17.

The image processing apparatus 16 has a function of supporting diagnosis by a doctor by performing determination related to diagnosis of a lesion in a case where a doctor or the like (hereinafter, simply referred to as a "doctor") performs diagnosis related to a lesion of the breast M using the radiation image.

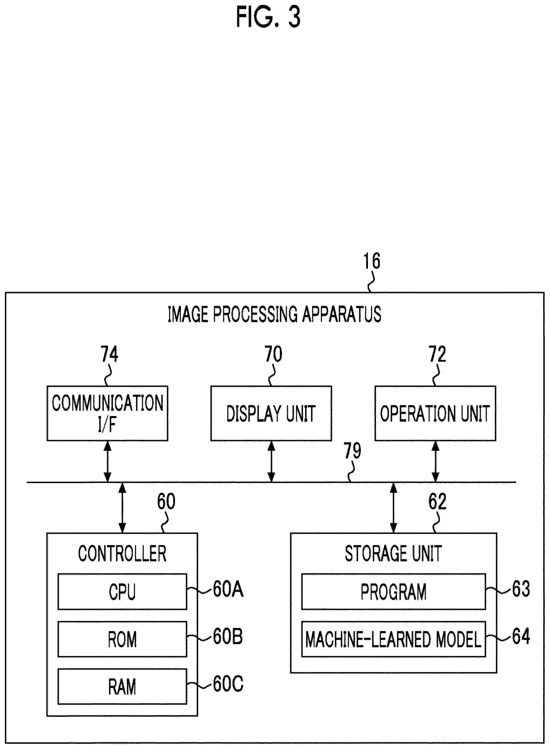

Fig. 3 illustrates an example of a configuration of the image processing apparatus 16. The image processing apparatus 16 includes a controller 60, a storage unit 62, a display unit 70, an operation unit 72, and a communication I/F 74. The controller 60, the storage unit 62, the display unit 70, the operation unit 72, and the communication I/F 74 are connected to each other via a bus 79 such as a system bus or a control bus such that various types of information can be exchanged.

The controller 60 controls overall operations of the image processing apparatus 16. The controller 60 is configured with a computer system including a CPU 60A, a ROM 60B, and a RAM 60C. Various programs, data, and the like for performing control by the CPU 60A are stored in advance in the ROM 60B. The RAM 60C temporarily stores various types of data.

The storage unit 62 is a non-volatile storage such as an HDD or an SSD. The storage unit 62 stores a program 63 for causing the controller 60 to execute various types of processing, a machine-learned model 64 for performing shape type determination processing to be described later, and the like.

The display unit 70 is a display that displays a radiation image, various types of information, and the like. The operation unit 72 is used to allow a doctor to input an instruction for diagnosing a lesion of a breast using a radiation image, various types of information, and the like. The operation unit 72 includes, for example, various switches, a touch panel, a touch pen, a mouse, and the like.

The communication I/F 74 performs communication of various types of information between the console 12 and the PACS 14 via the network 17 by wireless communication or wired communication.

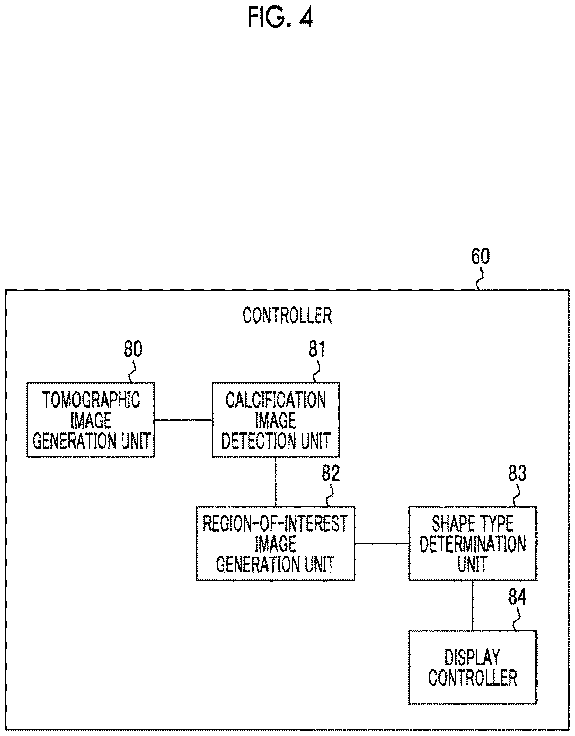

Fig. 4 illustrates an example of a function realized by the controller 60 of the image processing apparatus 16. The CPU 60A of the controller 60 realizes various functions by executing processing based on the program 63 stored in the storage unit 62. The controller 60 functions as a tomographic image generation unit 80, a calcification image detection unit 81, a region-of-interest image generation unit 82, a shape type determination unit 83, and a display controller 84.

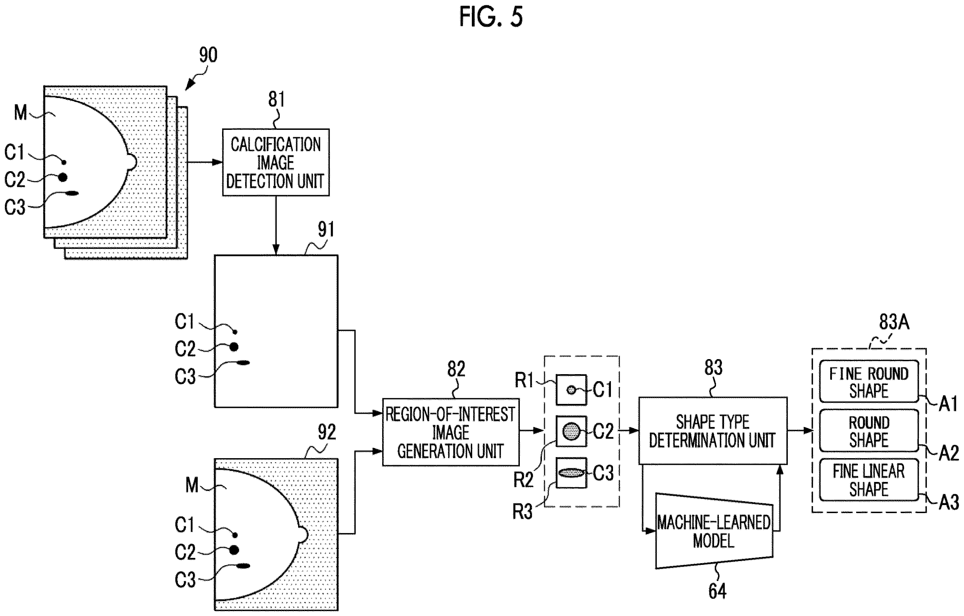

The tomographic image generation unit 80 has a function of generating a plurality of tomographic images 90 (refer to Fig. 5) from a series of the plurality of projection images. The tomographic image generation unit 80 acquires a series of the plurality of projection images from the console 12 of the mammography apparatus 10 or the PACS 14 based on an instruction for diagnosing a lesion. The tomographic image generation unit 80 generates a plurality of tomographic images 90 having different heights from the imaging surface 24A, from a series of the plurality of acquired projection images. For example, the tomographic image generation unit 80 generates a plurality of tomographic images 90 by reconfiguring a series of the plurality of projection images by a back projection method. As the back projection method, a filter back projection (FBP) method, a successive approximation reconfiguration method, or the like can be used. The tomographic image generation unit 80 outputs the plurality of generated tomographic images 90 to the calcification image detection unit 81.

Fig. 5 schematically illustrates a flow of processing by the image processing apparatus 16. Processing by the calcification image detection unit 81, the region-of-interest image generation unit 82, and the shape type determination unit 83 will be described with reference to Fig. 5.

The calcification image detection unit 81 performs calcification image detection processing of detecting a tissue image in which an occurrence of calcification is expected in the breast M (hereinafter, calcification image) based on the plurality of tomographic images 90 generated by the tomographic image generation unit 80. For example, the calcification image detection unit 81 generates a synthesized two-dimensional image based on the plurality of tomographic images 90, and performs detection processing using a known computer-aided diagnosis (CAD) algorithm based on the generated synthesized two-dimensional image. In the CAD algorithm, a probability (likelihood) indicating that a pixel in the synthesized two-dimensional image is a calcification image is derived, and a pixel of which the probability is equal to or higher than a predetermined threshold value is detected as the calcification image. There is a possibility that a position of the calcification image on the synthesized two-dimensional image and a position at which calcification occurs in the breast M may deviate from each other. For this reason, preferably, the calcification image detection unit 81 performs registration according to geometric or pattern matching.

The detection processing is not limited to the detection processing using the CAD algorithm. The calcification image detection unit 81 may perform detection processing using a machine-learned model obtained by performing machine learning. For example, the calcification image detection unit 81 may detect the calcification image by inputting the synthesized two-dimensional image generated based on the plurality of tomographic images 90 into the machine-learned model.

In addition, the calcification image detection unit 81 may detect the calcification image based on the plurality of tomographic images 90 without generating the synthesized two-dimensional image. For example, the calcification image detection unit 81 may detect the calcification image by inputting the plurality of tomographic images 90 into the machine-learned model. In addition, the calcification image detection unit 81 may perform detection processing (so-called rule-based detection processing) using a CAD algorithm based on the plurality of tomographic images 90.

The detection result of the calcification image by the calcification image detection unit 81 is output as, for example, a mask image 91 representing a position of the calcification image. The mask image 91 is a binary image in which a pixel included in the calcification image is represented by "1" and the other pixels are represented by "0". The calcification image detection unit 81 outputs one mask image 91 for the plurality of tomographic images 90. By performing the detection processing using the plurality of tomographic images 90, the calcification image can be detected with high detection accuracy. In the example illustrated in Fig. 5, three calcification images C1 to C3 are detected by the calcification image detection unit 81.

The region-of-interest image generation unit 82 generates a region-of-interest image (hereinafter, referred to as a region-of-interest (ROI) image) based on one projection image 92 among a series of the plurality of projection images used for the reconfiguration processing by the tomographic image generation unit 80 and the detection result of the calcification image by the calcification image detection unit 81. The projection image 92 used for generation of the ROI image by the region-of-interest image generation unit 82 is a projection image obtained at a position facing the detection surface 20A of the radiation detector 20 among a series of the plurality of projection images. The position facing the detection surface 20A is a position at which the irradiation angle α is 0 degree. In the present embodiment, the position facing the detection surface 20A is the irradiation position P4 illustrated in Fig. 2.

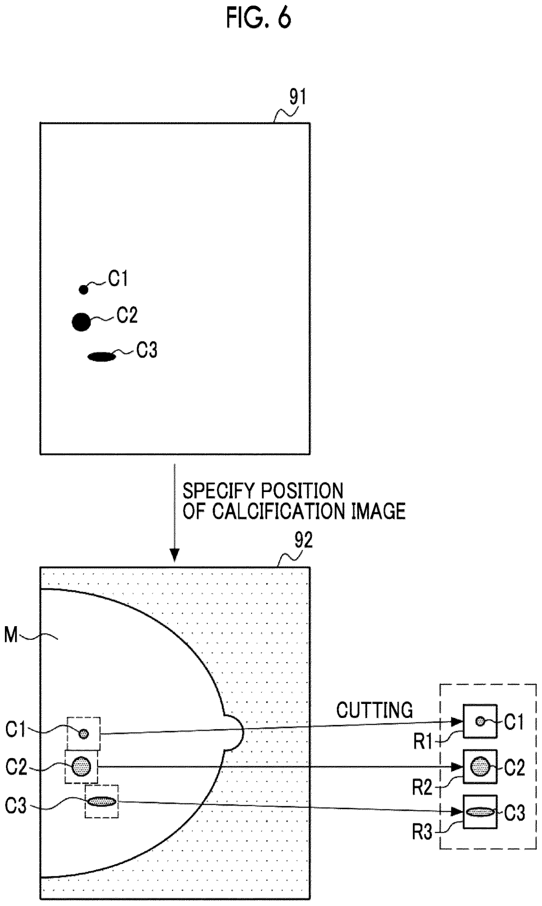

Fig. 6 conceptually illustrates region-of-interest image generation processing by the region-of-interest image generation unit 82. The region-of-interest image generation unit 82 generates a ROI image by cutting out a region including the calcification image from the projection image 92 based on the mask image 91. In addition, in a case where the plurality of calcification images are detected in the calcification image detection processing, the region-of-interest image generation unit 82 individually generates a ROI image for each of the plurality of calcification images. In the example illustrated in Fig. 6, a ROI image is individually generated for each of three calcification images C1 to C3. Thereby, a ROI image R1 including the calcification image C1, a ROI image R2 including the calcification image C2, and a ROI image R3 including the calcification image C3 are generated.

The shape type determination unit 83 performs shape type determination processing of determining a type of a shape of the calcification image included in the ROI image generated by the region-of-interest image generation processing. In the present embodiment, the shape type determination unit 83 inputs the ROI image to the machine-learned model 64 obtained by performing machine learning of a relationship between a calcification image and a type of a shape of the calcification image, and acquires a determination result 83A (hereinafter, referred to as a shape type determination result) that is output from the machine-learned model 64. The machine-learned model 64 is, for example, a convolutional neural network (CNN) obtained by performing machine learning by deep learning.

In the example illustrated in Fig. 5, the shape type determination unit 83 individually inputs each of the three ROI images R1 to R3 into the machine-learned model 64. From the machine-learned model 64, the shape type determination result 83A representing a type of a shape of the calcification image is output for each of the three ROI images R1 to R3. The shape type determination result 83A includes determination results A1 to A3. The determination result A1 represents that a type of a shape of the calcification image C1 included in the ROI image R1 is "fine round shape". The determination result A2 represents that a type of a shape of the calcification image C2 included in the ROI image R2 is "round shape". The determination result A3 represents that a type of a shape of the calcification image C3 included in the ROI image R3 is "fine linear shape".

In the present embodiment, the type of the shape of the calcification image includes not only a difference in shape but also a difference in size. The type of the shape of the calcification image is not limited to "fine round shape", " round shape", and "fine linear shape", and may include other shapes such as "linear shape". Preferably, the type of the shape of the calcification image is classified into classes such that whether the calcification image represents benignancy or malignancy can be determined.

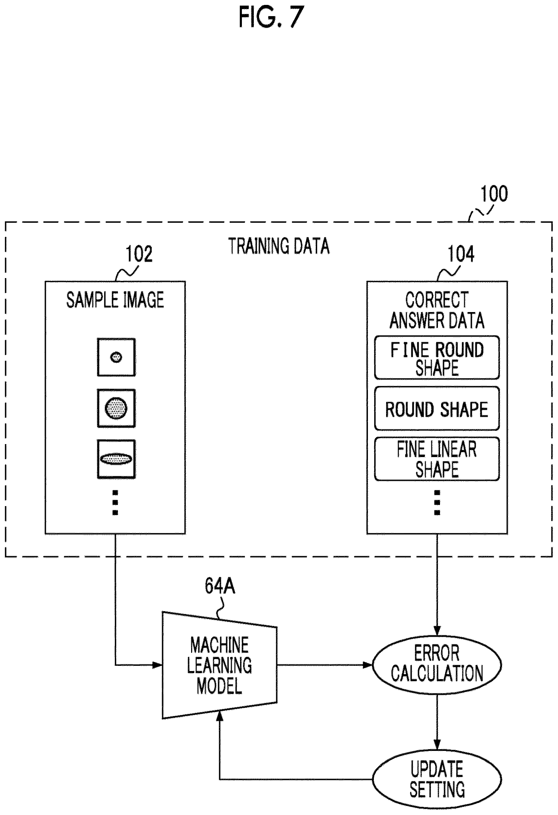

As illustrated in Fig. 7, the machine-learned model 64 is generated by performing machine learning on the machine learning model 64A using training data 100 in a learning phase. The training data 100 includes a set of a plurality of sample images 102 and a plurality of pieces of correct answer data 104. The sample image 102 is an image corresponding to the above-described ROI image, and includes a calcification image. The correct answer data 104 is information representing a correct answer of the type of the shape of the calcification image included in the sample image 102.

The machine learning is performed on the machine learning model 64A using, for example, an error back propagation method. In the learning phase, error calculation between the determination result obtained by inputting the sample image 102 to the machine learning model 64A and the correct answer data 104 and update setting of weights and biases are repeatedly performed. The machine learning model 64A on which machine learning is performed in the learning phase is stored in the storage unit 62, as a machine-learned model 64. The machine learning of the machine learning model 64A may be performed in the image processing apparatus 16 or in an external apparatus.

The display controller 84 performs display processing for displaying the shape type determination result 83A by the shape type determination processing on the display unit 70. Specifically, the display controller 84 highlights and displays the calcification image having a specific shape based on the shape type determination result 83A.

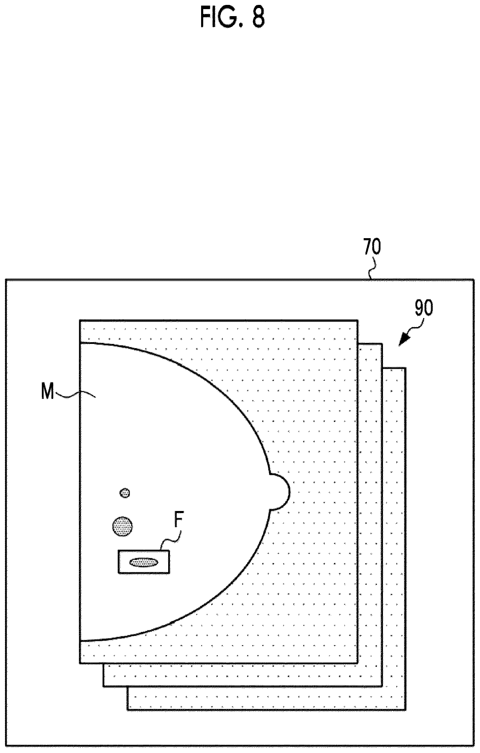

Fig. 8 illustrates an example of display processing by the display controller 84. For example, the display controller 84 displays the shape type determination result 83A together with the tomographic image 90 as a clinical image on the display unit 70. In the example illustrated in Fig. 8, the display controller 84 highlights and displays the calcification image having a shape (for example, a linear shape) representing a high degree of malignancy based on the shape type determination result 83A by surrounding the calcification image with a frame F. The displayed tomographic image 90 with the frame F is, for example, one tomographic image 90 selected from the plurality of tomographic images 90 via the operation unit 72.

In the example illustrated in Fig. 8, only the calcification image having a shape representing a high degree of malignancy is surrounded by the frame F. On the other hand, all the calcification images may be surrounded by frames F. In this case, colors of the frames F, line types of the frames F, and the like may be different based on the shape type determination result 83A. For example, the frame F surrounding the calcification image having a shape representing a high degree of malignancy is red, and the frame F surrounding the calcification image having a shape representing a low degree of malignancy is blue.

In addition, the highlight display is not limited to the form in which the calcification image is surrounded by the frame F. The color of the calcification image may be different based on the shape type determination result 83A. For example, the highlight display may be performed by coloring only the calcification image having a shape representing a high degree of malignancy. In addition, texts, symbols, and the like representing the shape type determination result 83A may be displayed on the display unit 70.

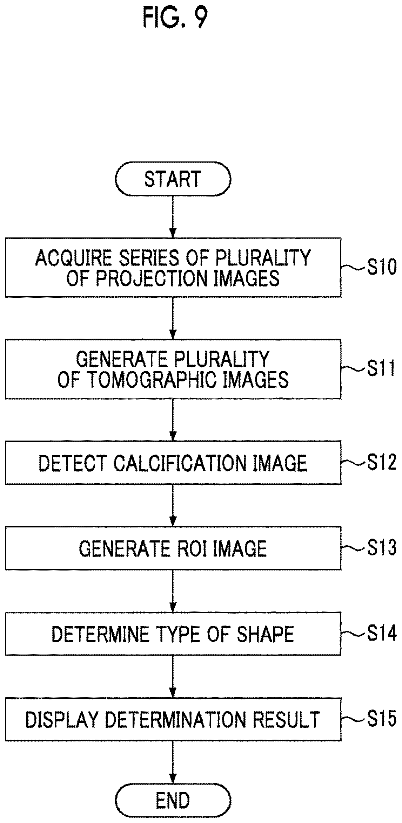

Next, a series of processing by the image processing apparatus 16 will be described with reference to Fig. 9. First, in step S10, the tomographic image generation unit 80 acquires a series of a plurality of projection images from the console 12 of the mammography apparatus 10 or the PACS 14.

In step S11, the tomographic image generation unit 80 generates a plurality of tomographic images 90 based on a series of the plurality of projection images acquired in step S10.

In step S12, the calcification image detection unit 81 detects a calcification image from the plurality of tomographic images 90 generated in step S11, and generates a mask image 91 as a detection result.

In step S13, the region-of-interest image generation unit 82 generates a ROI image by cutting out a region including the calcification image from the projection image 92 obtained at a position facing the detection surface 20A of the radiation detector 20, by using the mask image 91 generated in step S12.

In step S14, the shape type determination unit 83 determines a type of a shape of the calcification image included in the ROI image generated in step S13. Specifically, the shape type determination unit 83 inputs the ROI image to the machine-learned model 64, and acquires a shape type determination result 83A output from the machine-learned model 64.

In step S15, the display controller 84 performs display processing of displaying the shape type determination result 83A obtained in step S14 on the display unit 70. Specifically, the display controller 84 highlights and displays the calcification image having a shape representing a high degree of malignancy.

As described above, according to the technique of the present disclosure, the ROI image is generated from the projection image 92 obtained at the irradiation position facing the detection surface 20A of the radiation detector 20 among a series of the plurality of projection images. Thereby, it is possible to accurately determine the type of the shape of the calcification image. This is because that the shape of the calcification image is often not accurately represented in the synthesized two-dimensional image generated from the plurality of tomographic images and that the calcification image is blurred due to noise in the tomographic image and visibility is lowered.

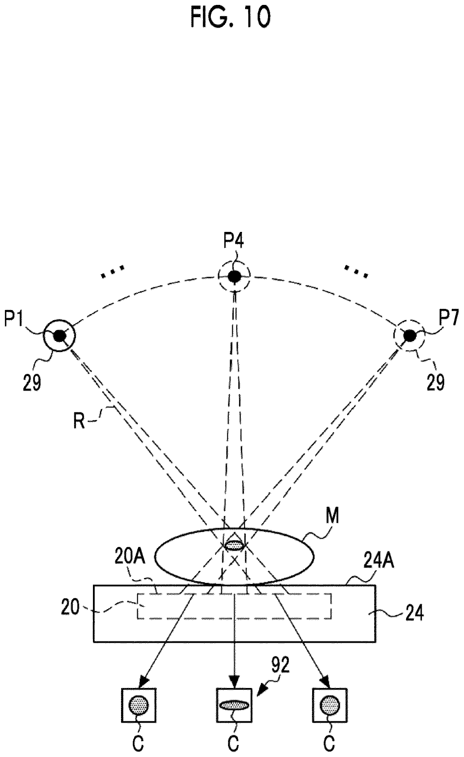

In addition, as illustrated in Fig. 10, among a series of the plurality of projection images obtained at the plurality of irradiation positions P1 to P7, in the projection image 92 obtained at the irradiation position P4 facing the detection surface 20A, the shape of the calcification image C is most accurately represented. Therefore, by using the ROI image generated from the projection image 92, it is possible to accurately determine the type of the shape of the calcification image.

In the embodiment, the irradiation position P4 is a position facing the detection surface 20A (that is, a position at which α = 0). On the other hand, the irradiation position may not exist at the position facing the detection surface 20A. In such a case, the ROI image may be generated using the projection image obtained at the irradiation position closest to the position facing the detection surface 20A among the plurality of irradiation positions at which a series of the plurality of projection images are obtained.

Further, in the embodiment, the calcification image detection unit 81 detects the calcification image from the plurality of tomographic images 90. The calcification image detection unit 81 may detect only a calcification image (so-called pale calcification image) of which a signal value is equal to or smaller than a certain value. This is because a shape of the pale calcification image is not accurately represented and it is difficult to determine a type of the shape on the tomographic image 90 as a clinical image that is displayed on the display unit 70.

Hereinafter, various modification examples of the embodiment will be described.

### [First Modification Example]

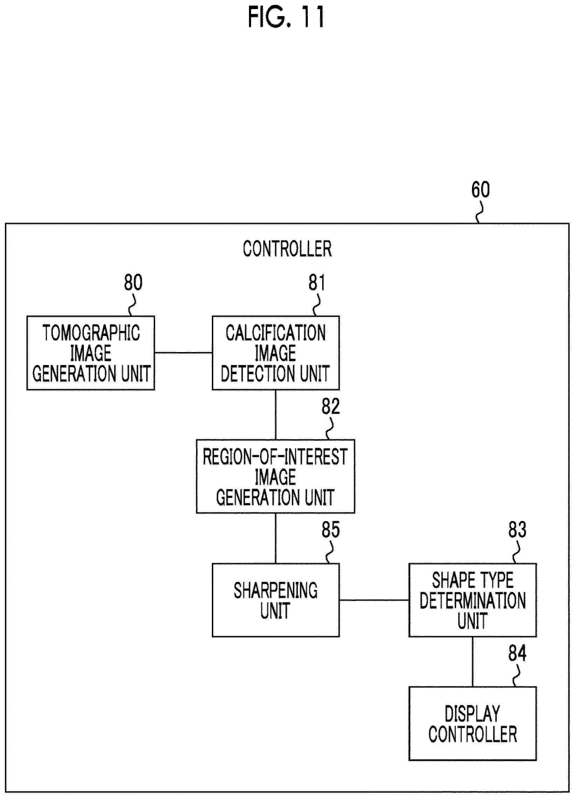

Fig. 11 illustrates a function realized by the controller 60 of the image processing apparatus 16 according to a first modification example. The first modification example is different from the embodiment in that the controller 60 functions as a sharpening unit 85 in addition to the tomographic image generation unit 80, the calcification image detection unit 81, the region-of-interest image generation unit 82, the shape type determination unit 83, and the display controller 84.

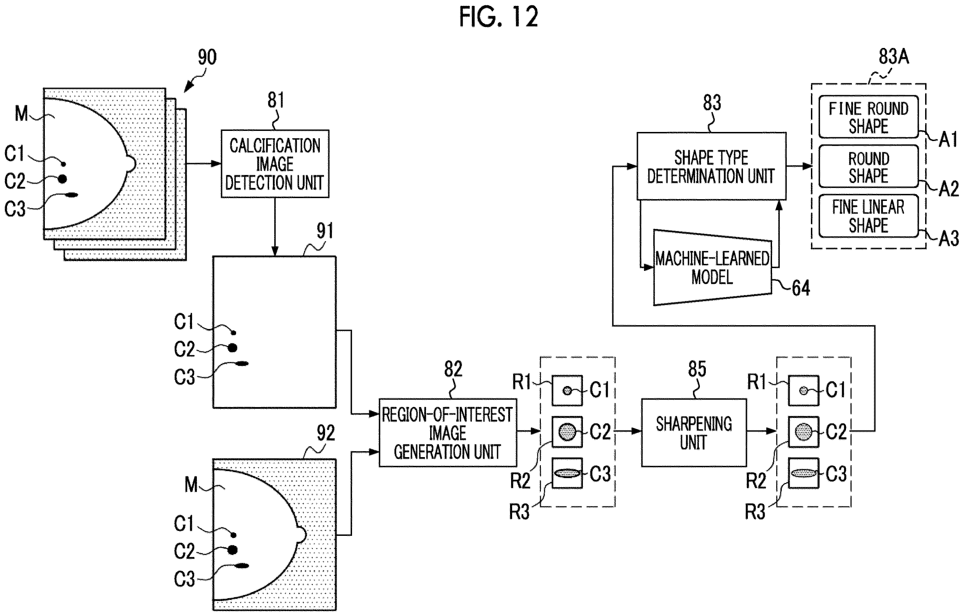

Fig. 12 schematically illustrates a flow of processing by the image processing apparatus 16 according to the first modification example. In the present modification example, the sharpening unit 85 performs noise removal processing or resolution enhancement processing, or both noise removal processing and resolution enhancement processing, on the ROI image generated by the region-of-interest image generation unit 82. Thereby, the ROI image is sharpened. For example, the sharpening unit 85 performs noise removal processing by well-known smoothing processing. In addition, for example, the sharpening unit 85 performs resolution enhancement by using a method described in JP2020-025786A. A resolution enhancement method described in JP2020-025786A is processing using a learned model obtained by performing machine learning of an input image such that a high-resolution image is output. The learned model outputs a high-resolution image by adding a new pixel between pixels of the input image and interpolating a pixel value of the added new pixel.

In the present modification example, the shape type determination unit 83 determines a type of a shape of the calcification image included in the ROI image sharpened by the sharpening unit 85. In this way, by using the sharpened ROI image, it is possible to more accurately determine the type of the shape of the calcification image.

### [Second Modification Example]

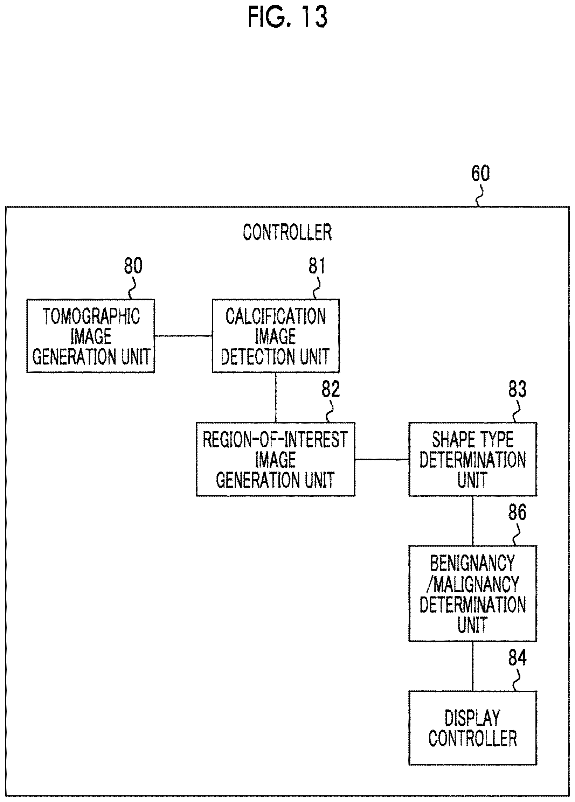

Fig. 13 illustrates a function realized by the controller 60 of the image processing apparatus 16 according to a second modification example. The second modification example is different from the embodiment in that the controller 60 functions as a benignancy/malignancy determination unit 86 in addition to the tomographic image generation unit 80, the calcification image detection unit 81, the region-of-interest image generation unit 82, the shape type determination unit 83, and the display controller 84.

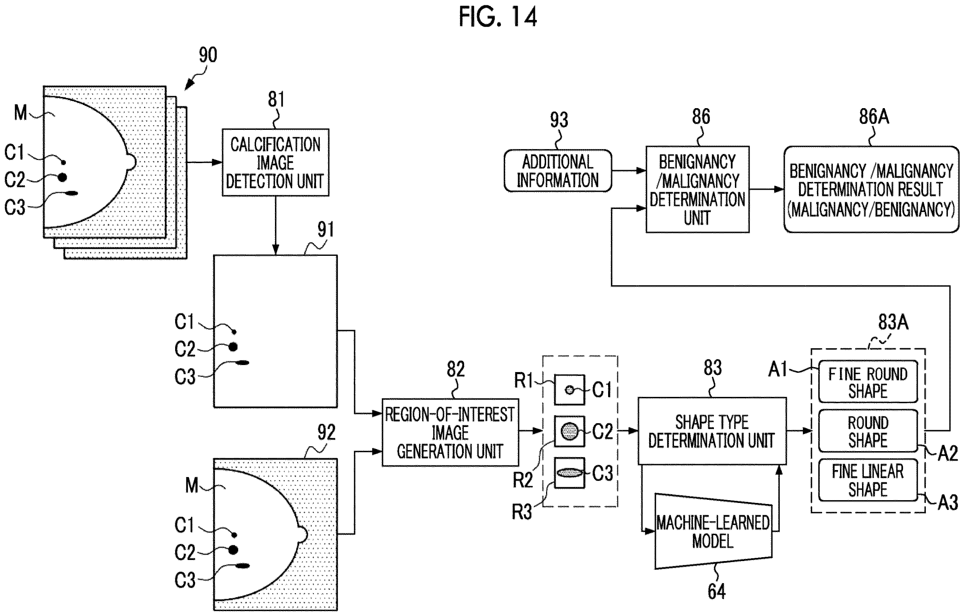

Fig. 14 schematically illustrates a flow of processing by the image processing apparatus 16 according to the second modification example. In the present modification example, the shape type determination result 83A output from the shape type determination unit 83 is input to the benignancy/malignancy determination unit 86. The benignancy/malignancy determination unit 86 determines whether the calcification image represents benignancy or malignancy, or determines a degree of malignancy represented by the calcification image, based on the shape type determination result 83A. In the example illustrated in Fig. 14, the benignancy/malignancy determination unit 86 determines whether the calcification image C1 represents benignancy or malignancy based on the determination result A1, determines whether the calcification image C2 represents benignancy or malignancy based on the determination result A2, and determines whether the calcification image C3 represents benignancy or malignancy based on the determination result A3.

In addition, additional information 93 other than the shape type determination result 83A may be input to the benignancy/malignancy determination unit 86. The additional information 93 is, for example, information such as a distribution of the calcification image, the calcification image in the synthesized two-dimensional image, and the like. The benignancy/malignancy determination unit 86 can accurately perform benignancy/malignancy determination by using the additional information 93 in addition to the shape type determination result 83A.

In the present modification example, the display controller 84 performs display processing of displaying the benignancy/malignancy determination result 86A output from the benignancy/malignancy determination unit 86 on the display unit 70. For example, the display controller 84 highlights and displays the calcification image determined as a malignancy based on the benignancy/malignancy determination result 86A. The display controller 84 may display a text, a symbol, or the like representing the benignancy/malignancy determination result 86A on the display unit 70.

As the benignancy/malignancy determination unit 86, for example, a determination device using a known CAD algorithm can be used. The benignancy/malignancy determination unit 86 may be configured by a machine-learned model obtained by performing machine learning.

### [Third Modification Example]

In the embodiment, the calcification image detection unit 81 is provided in the controller 60. On the other hand, in the technique of the present disclosure, the calcification image detection unit 81 is not necessarily provided, and the calcification image detection unit 81 may not be provided. In the third modification example, the region-of-interest image generation unit 82 generates a plurality of ROI images by dividing the projection image 92 obtained at a position facing the detection surface 20A of the radiation detector 20 into a plurality of regions.

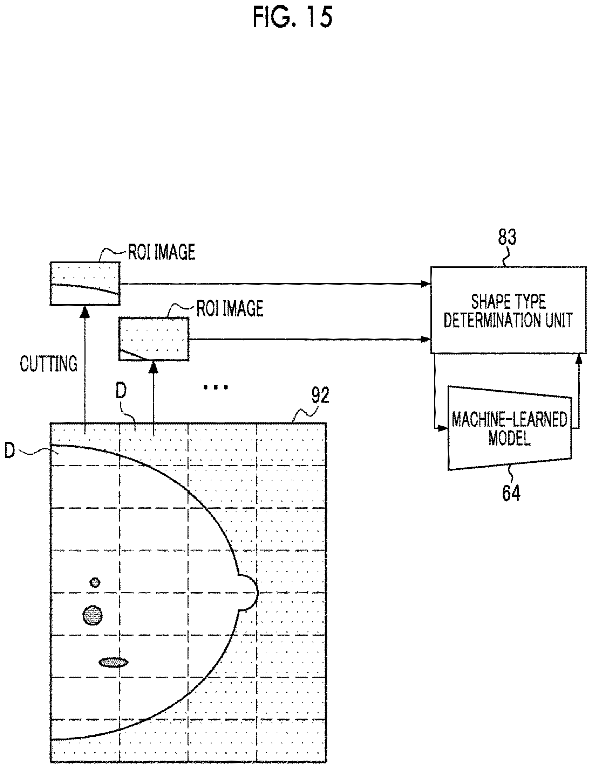

Fig. 15 illustrates an example of the region-of-interest image generation processing according to the third modification example. In the example illustrated in Fig. 15, the region-of-interest image generation unit 82 generates a plurality of ROI images by uniformly dividing the projection image 92 into a plurality of regions D and cutting out an image from each region D. That is, in the present modification example, the region-of-interest image generation unit 82 generates the ROI image by cutting out an image from the region D regardless of whether or not the calcification image is included in the region D. The region-of-interest image generation unit 82 individually inputs the plurality of generated ROI images to the shape type determination unit 83.

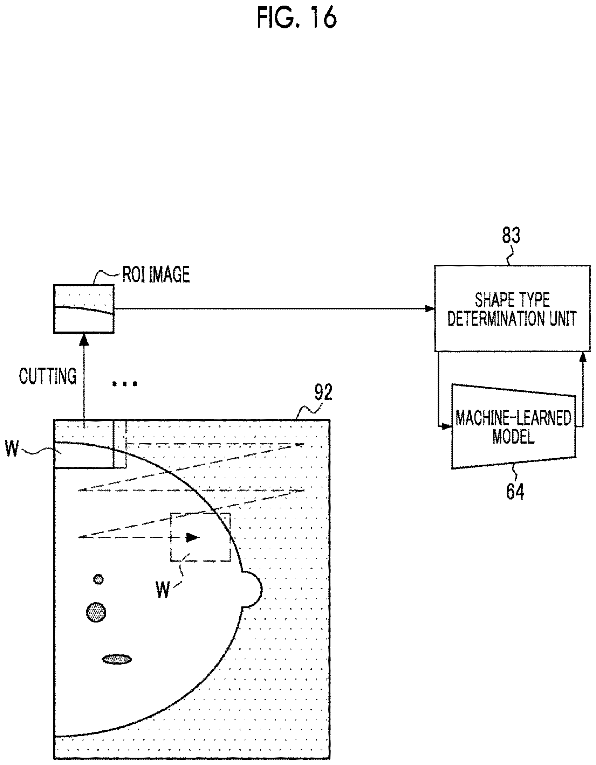

Fig. 16 illustrates another example of the region-of-interest image generation processing according to the third modification example. In the example illustrated in Fig. 16, the region-of-interest image generation unit 82 generates a plurality of ROI images by a sliding window method based on the projection image 92. Specifically, the region-of-interest image generation unit 82 generates the ROI images by setting a window W for the projection image 92 and cutting out an image from the window W while moving the window W by a small amount. The region-of-interest image generation unit 82 individually inputs the plurality of generated ROI images to the shape type determination unit 83.

As described above, in the present modification example, the region-of-interest image generation unit 82 generates a plurality of ROI images such that the entire region of the projection image 92 is covered. Therefore, it is possible to determine the type of the shape of the calcification image existing in the projection image 92 without omission.

### [Other Modification Examples]

In the embodiment and the modification examples, the region-of-interest image generation unit 82 generates the ROI image from the projection image 92 obtained at the irradiation position P4 facing the detection surface 20A of the radiation detector 20 among a series of the plurality of projection images acquired by tomosynthesis imaging. The technique of the present disclosure is not limited thereto, and the ROI image may be generated from the projection image obtained at the irradiation position P4 facing the detection surface 20A of the radiation detector 20 by normal imaging.

In addition, the technique of the present disclosure is not limited to tomosynthesis imaging, and can be applied to stereo imaging in which a subject is irradiated with radiations R from a plurality of irradiation positions having different irradiation angles α. That is, according to the technique of the present disclosure, a region-of-interest image is generated from a projection image, which is obtained at an irradiation position closest to a position facing a detection surface of a radiation detector, among a series of projection images obtained by irradiating a breast with radiations and imaging the breast.

The embodiment and the modification examples can be appropriately combined as long as there is no contradiction.

In addition, in the embodiment and the modification examples, as a hardware structure of a processing unit that executes various processing such as the tomographic image generation unit 80, the calcification image detection unit 81, the region-of-interest image generation unit 82, the shape type determination unit 83, the display controller 84, the sharpening unit 85, and the benignancy/malignancy determination unit 86, for example, various processors to be described below can be used. The various processors include a graphics processing unit (GPU) in addition to a CPU. In addition, the various processors are not limited to a general- purpose processor such as a CPU that functions as various processing units by executing software (program), and include a dedicated electric circuit, which is a processor having a circuit configuration specifically designed to execute specific processing, such as a programmable logic device (PLD) or an application specific integrated circuit (ASIC) that is a processor of which the circuit configuration may be changed after manufacturing such as a field programmable gate array (FPGA).

One processing unit may be configured by one of these various processors, or may be configured by a combination of two or more processors of the same type or different types (for example, a combination of a plurality of FPGAs or a combination of a CPU and an FPGA). Further, the plurality of processing units may be configured by one processor.

As an example in which the plurality of processing units are configured by one processor, firstly, as represented by a computer such as a client and a server, a form in which one processor is configured by a combination of one or more CPUs and software and the processor functions as the plurality of processing units may be adopted. Secondly, as represented by a system on chip (SoC) or the like, a form in which a processor that realizes the function of the entire system including the plurality of processing units by one integrated circuit (IC) chip is used may be adopted. As described above, the various processing units are configured by using one or more various processors as a hardware structure.

Further, as the hardware structure of the various processors, more specifically, an electric circuit (circuitry) in which circuit elements such as semiconductor elements are combined may be used.

In addition, in the embodiment and the modification examples, a form in which the program 63 is stored in the storage unit 62 in advance has been described. On the other hand, the present invention is not limited thereto. The program 63 may be provided by being recorded in a non-transitory recording medium such as a compact disc read only memory (CD-ROM), a digital versatile disc read only memory (DVD-ROM), or a Universal Serial Bus (USB) memory. Further, the program 63 may be downloaded from an external apparatus via a network.

The described contents and the illustrated contents are detailed explanations of a part according to the technique of the present disclosure, and are merely examples of the technique of the present disclosure. For example, the descriptions related to the configuration, the function, the operation, and the effect are descriptions related to examples of a configuration, a function, an operation, and an effect of a part according to the technique of the present disclosure. In order to avoid complications and facilitate understanding of the part according to the technique of the present disclosure, in the described contents and illustrated contents, descriptions of technical knowledge and the like that do not require particular explanations to enable implementation of the technique of the present disclosure are omitted.

### Explanation of References

2: radiography system
10: mammography apparatus
12: console
14: PACS
16: image processing apparatus
17: network
20: radiation detector
20A: detection surface
24: imaging table
24A: imaging surface
26: base
27: shaft portion
28: arm portion
29: radiation source
30: compression plate
32: compression unit
40: controller
42: storage unit
44: user I/F
46: communication I/F
50: storage unit
52: radiation image group
60: controller
62: storage unit
63: program
64: machine-learned model
64A: machine learning model
70: display unit
72: operation unit
74: communication I/F
79: bus
80: tomographic image generation unit
81: calcification image detection unit
82: region-of-interest image generation unit
83: shape type determination unit
83A: shape type determination result
84: display controller
85: sharpening unit
86: benignancy/malignancy determination unit
86A: benignancy/malignancy determination result
90: tomographic image
91: mask image
92: projection image
93: additional information
100: training data
102: sample image
104: correct answer data
α: irradiation angle
β: angle
A1, A2, A3: determination result
C, C1, C2, C3: calcification image
CL: normal line
D: region
F: frame
M: breast
Pk: irradiation position
R1, R2, R3: ROI image
R: radiation
RC: radiation axis
W: window

## Claims

1. An image processing apparatus comprising:
at least one processor (16),
wherein the processor is configured to execute
region-of-interest image generation processing of generating a region-of-interest image from a projection image, which is obtained at an irradiation position closest to a position facing a detection surface of a radiation detector, among a series of projection images obtained by irradiating a breast with radiations and imaging the breast, and
shape type determination processing of determining a type of a shape of a calcification image included in the region-of-interest image generated by the region-of-interest image generation processing.

2. The image processing apparatus according to claim 1,
wherein the processor is configured to:
execute calcification image detection processing of detecting the calcification image based on a plurality of tomographic images obtained from a plurality of the projection images; and
generate the region-of-interest image in the region-of-interest image generation processing by cutting out a region including the calcification image detected by the calcification image detection processing from the projection image obtained at the irradiation position closest to the position facing the detection surface.

3. The image processing apparatus according to claim 2,
wherein the processor is configured to individually generate the region-of-interest image for each of a plurality of the calcification images in the region-of-interest image generation processing in a case where the plurality of calcification images are detected in the calcification image detection processing.

4. The image processing apparatus according to claim 2 or 3,
wherein the processor is configured to detect only the calcification image of which a signal value is equal to or smaller than a certain value in the calcification image detection processing.

5. The image processing apparatus according to claim 1,
wherein the processor is configured to generate a plurality of the region-of-interest images by dividing the projection image obtained at the irradiation position closest to the position facing the detection surface into a plurality of regions in the region-of-interest image generation processing.

6. The image processing apparatus according to any one of claims 1 to 5,
wherein the processor is configured to execute the shape type determination processing by inputting the region-of-interest image into a machine-learned model obtained by performing machine learning of a relationship between the calcification image and the type of the shape of the calcification image.

7. The image processing apparatus according to any one of claims 1 to 6,
wherein the processor is configured to determine the shape of the calcification image after performing noise removal processing or resolution enhancement processing, or both the noise removal processing and the resolution enhancement processing on the region-of-interest image in the shape type determination processing.

8. The image processing apparatus according to any one of claims 1 to 7,
wherein the processor is configured to execute display processing of displaying a shape type determination result by the shape type determination processing on a display unit.

9. The image processing apparatus according to claim 8,
wherein the processor is configured to highlight and display the calcification image having a specific shape based on the shape type determination result in the display processing.

10. The image processing apparatus according to any one of claims 1 to 9,
wherein the processor is configured to determine whether the calcification image represents benignancy or malignancy, or determine a degree of malignancy represented by the calcification image, based on a shape type determination result by the shape type determination processing.

11. An image processing method comprising:
a region-of-interest image generation step of generating a region-of-interest image from a projection image, which is obtained at an irradiation position closest to a position facing a detection surface of a radiation detector, among a series of projection images obtained by irradiating a breast with radiations and imaging the breast; and
a shape type determination step of determining a type of a shape of a calcification image included in the region-of-interest image generated by the region-of-interest image generation step.

12. A computer-readable storage medium storing a program causing a computer to execute a process comprising:
region-of-interest image generation processing of generating a region-of-interest image from a projection image, which is obtained at an irradiation position closest to a position facing a detection surface of a radiation detector, among a series of projection images obtained by irradiating a breast with radiations and imaging the breast; and
shape type determination processing of determining a type of a shape of a calcification image included in the region-of-interest image generated by the region-of-interest image generation processing.

## Patentansprüche

1. Bildverarbeitungsgerät aufweisend:
mindestens einen Prozessor (16),
wobei der Prozessor so konfiguriert ist, dass er Folgendes ausführt
Bild-eines-interessierenden-Bereichs-Erzeugungsverarbeitung zur Erzeugung eines Bildes des interessierenden Bereichs aus einem Projektionsbild, das an einer Bestrahlungsposition erhalten wird, die am nächsten zu einer Position liegt, die einer Erfassungsfläche eines Strahlungsdetektors gegenüberliegt, aus einer Reihe von Projektionsbildern, die durch Bestrahlung einer Brust mit Strahlen und Abbilden der Brust erhalten werden, und
Formtypbestimmungsverarbeitung zur Bestimmung eines Typs einer Form eines Verkalkungsbildes, das in dem von der Bildgenerierungsverarbeitung für den Bereich von Interesse erzeugten Bild enthalten ist.

2. Bildverarbeitungsgerät nach Anspruch 1,
wobei der Prozessor konfiguriert ist zum:
Ausführen einer Verkalkungsbilderkennungsverarbeitung zum Erkennen des Verkalkungsbildes auf der Grundlage einer Vielzahl von tomographischen Bildern, die aus einer Vielzahl von Projektionsbildern erhalten wurden; und
Erzeugen des Bildes des interessierenden Bereichs in der Bild-eines-interessierenden-Bereichs-Erzeugungsverarbeitung durch Ausschneiden eines Bereichs, der das durch die Verkalkungsbilderkennungsverarbeitung erkannte Bild enthält, aus dem Projektionsbild, das an der Bestrahlungsposition erhalten wurde, die der der Erfassungsfläche zugewandten Position am nächsten liegt.

3. Bildverarbeitungsgerät nach Anspruch 2,
wobei der Prozessor so konfiguriert ist, dass er das Bild des interessierenden Bereichs für jedes einer Vielzahl von Verkalkungsbildern in der Bild-eines-interessierenden-Bereichs-Erzeugungsverarbeitung individuell erzeugt, wenn die Vielzahl von Verkalkungsbildern in der Verkalkungsbilderkennungsverarbeitung erkannt wird.

4. Bildverarbeitungsgerät nach Anspruch 2 oder 3,
wobei der Prozessor so konfiguriert ist, dass er nur das Verkalkungsbild erkennt, dessen Signalwert gleich oder kleiner als ein bestimmter Wert bei der Verkalkungsbilderkennungsverarbeitung ist.

5. Bildverarbeitungsgerät nach Anspruch 1,
wobei der Prozessor so konfiguriert ist, dass er eine Vielzahl von Bildern des interessierenden Bereichs erzeugt, indem er das Projektionsbild, das an der Bestrahlungsposition erhalten wurde, die der der Erfassungsfläche zugewandten Position am nächsten liegt, in eine Vielzahl von Bereichen in der Bilderzeugungsverarbeitung des interessierenden Bereichs unterteilt.

6. Bildverarbeitungsgerät nach einem der Ansprüche 1 bis 5,
wobei der Prozessor so konfiguriert ist, dass er die Formbestimmungsverarbeitung durch Eingabe des Bildes des interessierenden Bereichs in ein maschinell erlerntes Modell ausführt, das durch Durchführen von maschinellem Lernen einer Beziehung zwischen dem Verkalkungsbild und dem Typ der Form des Verkalkungsbildes erhalten wird.

7. Bildverarbeitungsgerät nach einem der Ansprüche 1 bis 6,
wobei der Prozessor so konfiguriert ist, dass er die Form des Verkalkungsbildes bestimmt, nachdem er eine Rauschentfernungsverarbeitung oder eine Auflösungsverbesserungsverarbeitung oder sowohl die Rauschentfernungsverarbeitung als auch die Auflösungsverbesserungsverarbeitung an dem Bild des interessierenden Bereichs in der Formtypbestimmungsverarbeitung durchgeführt hat.

8. Bildverarbeitungsgerät nach einem der Ansprüche 1 bis 7,
wobei der Prozessor so konfiguriert ist, dass er eine Anzeigeverarbeitung zum Anzeigen eines Ergebnisses der Formtypbestimmung durch die Formtypbestimmungsverarbeitung auf einer Anzeigeeinheit ausführt.

9. Bildverarbeitungsgerät nach Anspruch 8,
wobei der Prozessor so konfiguriert ist, dass er das Verkalkungsbild mit einer bestimmten Form auf der Grundlage des Ergebnisses der Formtypbestimmung bei der Anzeigeverarbeitung hervorhebt und anzeigt.

10. Bildverarbeitungsgerät nach einem der Ansprüche 1 bis 9,
wobei der Prozessor konfiguriert ist, um zu bestimmen, ob das Verkalkungsbild gutartig oder bösartig ist, oder um einen Grad der Bösartigkeit zu bestimmen, der durch das Verkalkungsbild dargestellt wird, basierend auf einem Formtypbestimmungsergebnis durch die Formtypbestimmungsverarbeitung.

11. Bildverarbeitungsverfahren, das Folgendes umfasst:
einen Bild-eines-interessierenden-Bereichs-Erzeugungsschritt, bei dem ein Bild des interessierenden Bereichs aus einem Projektionsbild erzeugt wird, das an einer Bestrahlungsposition erhalten wird, die einer Position am nächsten liegt, die einer Erfassungsfläche eines Strahlungsdetektors zugewandt ist, aus einer Reihe von Projektionsbildern, die durch Bestrahlen einer Brust mit Strahlen und Abbildung der Brust erhalten werden; und
einen Formtypbestimmungsschritt zum Bestimmen eines Typs einer Form eines Verkalkungsbildes, das in dem durch den Bild-eines-interessierenden-Bereichs-Erzeugungsschritt erzeugten Bildes des interessierenden Bereichs enthalten ist.

12. Computerlesbares Speichermedium, das ein Programm speichert, das einen Computer veranlasst, einen Prozess auszuführen, der Folgendes umfasst:
Bild-eines-interessierenden-Bereichs-Erzeugungsverarbeitung zur Erzeugung eines Bildes des interessierenden Bereichs aus einem Projektionsbild, das an einer Bestrahlungsposition erhalten wird, die am nächsten zu einer Position liegt, die einer Erfassungsfläche eines Strahlungsdetektors zugewandt ist, aus einer Reihe von Projektionsbildern, die durch Bestrahlen einer Brust mit Strahlen und Abbildung der Brust erhalten werden; und
Formtypbestimmungsverarbeitung zur Bestimmung eines Typs einer Form eines Verkalkungsbildes, das in dem von der Bildgenerierungsverarbeitung für den interessierenden Bereich erzeugten Bild enthalten ist.

## Revendications

1. Appareil de traitement d'images comprenant :
au moins un processeur (16),
dans lequel le processeur est configuré pour exécuter
traitement de génération d'image de région d'intérêt consistant à générer une image de région d'intérêt à partir d'image de projection obtenue à une position d'irradiation la plus proche d'une position faisant face à une surface de détection d'un détecteur de rayonnement, parmi une série d'images de projection obtenues par irradiation d'un sein avec des rayonnements et par imagerie du sein, et
traitement de détermination du type de forme consistant à déterminer le type de forme d'image de calcification incluse dans l'image de la région d'intérêt générée par le traitement de génération d'image de la région d'intérêt.

2. Appareil de traitement d'images selon la revendication 1,
dans lequel le processeur est configuré pour :
exécuter traitement de détection d'image de calcification consistant à détecter l'image de calcification sur la base d'une pluralité d'images tomographiques obtenues à partir d'une pluralité d'images de projection ; et
générer l'image de la région d'intérêt dans le traitement de génération d'image de la région d'intérêt en découpant une région comprenant l'image de calcification détectée par traitement de détection d'image de calcification à partir de l'image de projection obtenue à la position d'irradiation la plus proche de la position faisant face à la surface de détection.

3. Appareil de traitement d'images selon la revendication 2,
le processeur est configuré pour générer individuellement l'image de la région d'intérêt pour chacune des images de calcification dans le traitement de génération d'image de région d'intérêt dans le cas où la pluralité d'images de calcification est détectée dans traitement de détection d'image de calcification.

4. Appareil de traitement d'images selon la revendication 2 ou 3,
le processeur est configuré pour ne détecter que l'image de calcification dont la valeur du signal est égale ou inférieure à une certaine valeur dans traitement de détection d'image de calcification.

5. Appareil de traitement d'images selon la revendication 1,
le processeur est configuré pour générer une pluralité d'images de régions d'intérêt en divisant l'image de projection obtenue à la position d'irradiation la plus proche de la position faisant face à la surface de détection en une pluralité de régions dans le traitement de génération d'image de région d'intérêt.

6. Appareil de traitement d'images selon l'une des revendications 1 à 5,
le processeur est configuré pour exécuter le traitement de détermination du type de forme en introduisant l'image de la région d'intérêt dans un modèle d'apprentissage automatique obtenu en effectuant un apprentissage automatique d'une relation entre l'image de calcification et le type de forme de l'image de calcification.

7. Appareil de traitement d'images selon l'une des revendications 1 à 6,
le processeur est configuré pour déterminer la forme de l'image de calcification après avoir effectué un traitement de suppression du bruit ou un traitement d'amélioration de la résolution, ou à la fois le traitement de suppression du bruit et le traitement d'amélioration de la résolution sur l'image de la région d'intérêt dans le traitement de détermination du type de forme.

8. Appareil de traitement d'images selon l'une des revendications 1 à 7,
le processeur est configuré pour exécuter le traitement d'affichage d'un résultat de détermination de type de forme par le traitement de détermination du type de forme sur une unité d'affichage.

9. Appareil de traitement d'images selon la revendication 8,
le processeur est configuré pour mettre en évidence et afficher l'image de calcification ayant une forme spécifique sur la base du résultat de la détermination du type de forme dans le traitement de l'affichage.

10. Appareil de traitement d'images selon l'une des revendications 1 à 9,
le processeur est configuré pour déterminer si l'image de calcification est bénigne ou maligne, ou pour déterminer le degré de malignité représenté par l'image de calcification, sur la base d'un résultat de détermination du type de forme obtenu par le traitement de détermination du type de forme.

11. Méthode de traitement d'images comprenant
une étape de génération d'image de région d'intérêt consistant à générer une image de région d'intérêt à partir d'image de projection obtenue à une position d'irradiation la plus proche d'une position faisant face à une surface de détection d'un détecteur de rayonnement, parmi une série d'images de projection obtenues par irradiation d'un sein avec des rayonnements et par imagerie du sein ; et
une étape de détermination du type de forme consistant à déterminer le type de forme d'image de calcification incluse dans l'image de la région d'intérêt générée par l'étape de génération d'image de la région d'intérêt.

12. Support de stockage lisible par ordinateur stockant un programme amenant un ordinateur à exécuter un processus comprenant :
traitement de génération d'image de région d'intérêt consistant à générer une image de région d'intérêt à partir d'image de projection, qui est obtenue à une position d'irradiation la plus proche d'une position faisant face à une surface de détection d'un détecteur de rayonnement, parmi une série d'images de projection obtenues en irradiant un sein avec des rayonnements et en réalisant une imagerie du sein ; et
le traitement de détermination du type de forme consistant à déterminer le type de forme d'image de calcification incluse dans l'image de la région d'intérêt générée par le traitement de génération d'image de la région d'intérêt.
